# EUROPEAN PATENT APPLICATION

(11) **EP 3 942 926 A1**
(43) Date of publication of application: **26.01.2022**
(21) Application number: 20186947.6
(22) Date of filing: 21.07.2020
(51) Int. Cl.: A01K 29/00, A01K 15/02, A61B 5/024

(54) **SENSOR DEVICE AND METHOD FOR HORSE MONITORING**

(71) Applicant: Ruxbury ApS, 2970 Hørsholm (DK)
(72) Inventor: GOLDMAN, Tomasz, 2970 Hørsholm (DK)
(74) Representative: Aera A/S

(57) **Abstract**

A method for monitoring a horse is disclosed, the method comprising positioning a sensor device (300) below an ear of the horse (1), the sensor device comprising a sensor unit (303) comprising a first optical sensor (303A); obtaining sensor data from the sensor unit, the sensor data comprising first sensor data from the first optical sensor; determining, with the sensor device, horse data of the horse based on the sensor data; and transmitting the horse data to a monitor device (400).

## Description

The present disclosure relates to a method for horse monitoring, to a sensor device for a horse, a horse head gear, and a monitoring system.

### BACKGROUND

Horses may be subject to several distress situations, where the presence of a caregiver may be vital. For example, colic is one of the most dangerous and costly equine medical problems. Colic is a symptom of a disease and may result in abdominal pain for the horse. The disease, if left untreated, may be fatal for the horse. Further, another distress situation for horses may be if a horse is cast in a stall, which may be a cause of serious injury. Furthermore, foaling when a mare gives birth (to a foal) is another event where the presence of a caregiver may reduce risks to the foal and the mare and prevent complications.

Those events and distress situations may occur at remote locations (for example when the horse is the stall or in a field, far from the owner or caretakers), and often at night.

It is therefore a challenge for horse owners and caretakers to avoid and prevent these events.

Sensor devices and monitoring systems for horses exist which allow horse owners or horse personnel to monitor their horses. However, sensor devices and monitoring systems for horses may be cumbersome for the horse and the horse equipment and also complicated to install (e.g. mount) on the horse. Often, the horse monitoring systems comprise horse equipment that may be uncomfortable for the horse (such as causing injuries to the horse), which even may have to be removed from the horse (e.g. before going into the stall), and can therefore not be used for monitoring the horse at all time (such as in the stall).

Solutions for monitoring horses exists, where for example fitness products which have been adapted for horses, which use electrocardiogram, ECG, measurement methods to monitor a heart rate of the horse. However, it may not be advisable to use an abdominal belt with sensors, for long time unattended monitoring.

### SUMMARY

There is a need for methods for monitoring horses, sensor devices, horse head gear, and monitoring systems which mitigate, alleviate or address the shortcomings existing and provide an improved monitoring of horses including one or more of improved continuous monitoring, improved positioning of the sensor device, improved reliability of measurement, improved comfort for the horse, simpler and more flexible mounting of the sensor device, and improved power consumption.

A method for monitoring a horse is provided. The method comprises positioning a sensor device below an ear of the horse, the sensor device comprising a sensor unit comprising a first optical sensor. The method comprises obtaining sensor data from the sensor unit, the sensor data comprising first sensor data from the first optical sensor. The method comprises determining, with the sensor device, horse data of the horse based on the sensor data. The method comprises transmitting the horse data to a monitor device.

Further, a sensor device is provided. The sensor device comprises a sensor device housing, a memory, a processor, and a sensor unit comprising a first optical sensor connected to the processor. The sensor device is configured to be positioned below an ear of a horse. The processor is configured to obtain sensor data from the sensor unit, the sensor data comprising first sensor data from the first optical sensor. The processor is configured to determine horse data of the horse based on the sensor data. The processor is configured to transmit the horse data to a monitor device.

Further, a horse head gear is provided, the horse head gear comprising a horse head equipment and a sensor device according to this disclosure.

Further, a monitoring system is provided, the monitoring system comprising a sensor device according to this disclosure and a monitor device for monitoring the sensor device.

It is an advantage of the present disclosure that an improved monitoring of horses is provided.

It may be appreciated that the present disclosure provides an improved positioning of the sensor device, which in turn provides improved reliability of measurement including improved precision of measurement, thereby improving the detection of distress situations.

The present disclosure allows for improved comfort for the horse by keeping horse personnel informed of the horse's condition and in particular facilitates fast reactions of the horse personnel to potentially dangerous conditions of the horse. For example, positioning the sensor device below an ear of the horse may be advantageous since the thickness of the skin of the horse is smaller than at other places of the body of the horse. Further, it may be advantageous to position the sensor device below the ear of the horse since the amount of fur at that position may be less than at other places of the body of the horse.

Further, it is an advantage of the present disclosure that a simpler and more flexible mounting of the sensor device is provided. The present disclosure provides a sensor device that may be mounted on existing horse head equipment.

It may be appreciated that the present disclosure provides horse monitoring with an improved or reduced power consumption which allows for long-term monitoring of the horse and reduces the need for time-consuming recharging and/or exchange of batteries.

### BRIEF DESCRIPTION OF THE DRAWINGS

The above and other features and advantages of the present invention will become readily apparent to those skilled in the art by the following detailed description of exemplary communication devices, methods, and/or server devices thereof with reference to the attached drawings, in which:
Fig. 1 is a schematic representation illustrating an example scenario of a sensor device according to the disclosure, positioned below an ear of a horse,
Fig. 2 is a schematic representation illustrating an example scenario of a sensor device according to the disclosure, positioned below an ear of a horse at a sensor area,
Fig. 3 is a flow diagram of an exemplary method according to the disclosure,
Fig. 4 is a block diagram illustrating a sensor device according to the disclosure,
Figs. 5A-5B are perspective views of an example sensor device according to the disclosure seen respectively from the proximal side and the distal side.

### DETAILED DESCRIPTION

Various exemplary methods and/or sensor devices and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the methods and/or sensor devices. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other methods and/or sensor devices even if not so illustrated, or if not so explicitly described.

In the following, whenever referring to proximal side or surface of a layer, an element, a device or part of a device, the referral is to the fur or skin-facing side or surface of the horse, when a horse wears the sensor device. Likewise, whenever referring to the distal side or surface of a layer, an element, a device or part of a device, the referral is to the side or surface facing away from the horse fur, when a horse wears the sensor device. In other words, the proximal side or surface is the side or surface closest to the horse, when the sensor device is fitted (such as mounted) on a horse head equipment and the distal side is the opposite side or surface - the side or surface furthest away from the horse in use.

The axial direction is defined as the direction of the sensor device, when a horse wears the sensor device. Thus, the axial direction is generally perpendicular to fur or skin-facing side or surface of the horse.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the sensor device than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

A method, for monitoring a horse is disclosed. The method comprises positioning a sensor device below an ear of the horse, the sensor device comprising a sensor unit comprising a first optical sensor. In one or more methods and/or sensor devices, positioning the sensor device below an ear of the horse comprises to position the sensor device in or at a sensor area. The sensor area may be representative of an area below the ear of the horse where the sensor device may be positioned. The term below the ear of the horse may be understood as between the ear of the horse and a temporal bone of the horse, e.g. substantially at the skin surface for example where an arteria temporalis superficialis is located under the skin. The term below the ear of the horse may be understood as within a distance of 50 cm from the ear of the horse. In other words, positioning a sensor device below an ear of the horse may comprise positioning the sensor device within a distance of 50 cm, such as within 20 cm, from the ear of the horse. The sensor area may overlap a part of the mandible of the horse and/or a part of the temporal bone of the horse. In other words, the sensor area may be said to be on the side of the head of the horse. The area below the ear of the horse may be rich in arterial blood (for example close to the arteria temporalis superficialis). The first optical sensor may be configured to be at or on the proximal surface, such as in contact with the proximal surface or at least within 1 cm from the proximal surface. By positioning the sensor device on the proximal surface and/or below the ear of the horse, an improved reliability and stability of measurements is achieved. An advantage of positioning the sensor device below the ear of the horse may be that the area below the ear of the horse may be rich in arterial blood (for example close to the arteria temporalis superficialis) and the skin and/or fur of the horse may be thinner. The precision of measurement of the sensor device and the detection of distress situations may be improved, and in turn also improving the reliability of measurement of the sensor device. Furthermore, by positioning the sensor device below the ear of the horse, an improved comfort may be provided for the horse, for example substantially avoiding that the horse notices the sensor device and is disturbed. Further, by positioning the sensor device below the ear of the horse, the sensor device may be mounted, e.g. releasably mounted, on existing horse head equipment. This may allow to use the sensor device with each horse's own equipment, instead of having to provide special equipment that may be difficult to fit on all horses. This may allow an easier mounting of the sensor device with less fitting to each horse for the users.

Horses may have a natural recess below the ear at the temporal bone or above the temporal bone, where it may be advantageous to position the sensor device (for example such that the sensor device may be stabilized, e.g. improving the measurement of the sensor device).

The method comprises obtaining sensor data from the sensor unit, the sensor data comprising first sensor data from the first optical sensor. The first sensor data may comprise and/or be based on one or more first optical signals (such as one or more optical counts measured by the first optical sensor over time). The first optical sensor may comprise one or more optical sources, such as one or more transmitters or emitters of optical signals, for example photo emitters, e.g. LEDs photodiodes. The first optical sensor may comprise one or more optical receivers (such as photo detectors). The first optical sensor may be configured to measure one or more of a heart-rate, a cardiac cycle, a respiration, and a blood pressure of the horse.

In one or more exemplary methods and/or sensor devices, the sensor unit comprises a second sensor and the sensor data comprises second sensor data from the second sensor. The second sensor may be a motion sensor and the second sensor data may be indicative of head motion of the horse.

The method comprises determining e.g. using the processor, with the sensor device, horse data of the horse based on the sensor data. In one or more exemplary methods, determining horse data of the horse based on the sensor data, may comprise determining the horse data based on the first sensor data. For example, determining the horse data may comprise determining a heart rate (such as a pulse) of the horse based on (first) sensor data, e.g. based on one or more first optical signals (such as one or more optical counts measured by the first optical sensor over time). For example, determining the horse data may comprise determining one or more of a heart-rate, a cardiac cycle, a pulse, and a blood pressure of the horse. In one or more embodiments, determining the horse data comprises to process the sensor data. In other words, the horse data may comprise one or more of a heart-rate, a cardiac cycle, a pulse, a respiration, and a blood pressure of the horse.

In the present context, horse data may comprise one or more of cardiac data and motion data. Cardiac data may comprise heart rate data, such as heart rate, e.g. heart rate averaged over a time period. Motion data comprise a motion index and/or a gait type. The motion index may be an integer optionally on an arbitrary scale, such as between zero and four. A motion index with a low value may be indicative of low activity and/or a motion index with a high value may be value may be indicative of high activity. The gait type may be selected from a plurality of gait types, such as from a plurality of walk, trot, gallop, canter, and tølt (tölt). The plurality of gait types may include standing and/or resting, such as standing still and/or laying down. The gait type may be selected from at least three different gait types, e.g. including gallop and/or canter. In other words, determining horse data of the horse based on the sensor data may comprise determining cardiac data and include the cardiac data in the horse data. Further, determining horse data of the horse based on the sensor data may comprise determining motion data, such as a motion index and/or a gait type and include the motion data in the horse data.

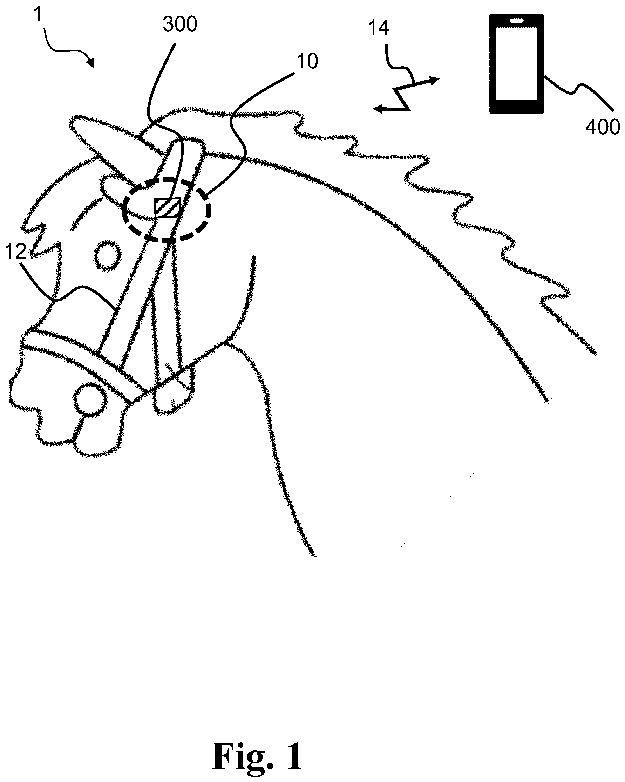

The method comprises transmitting, e.g. via the first interface, the horse data to a monitor device. The monitor device (such as the monitor device 400 of Fig. 1) may comprise one or more of: an electronic device (such as a wireless electronic device), a mobile phone, a smartphone, and/or a computer. The transmission of the horse data may be performed directly to the monitor device or may occur through a relay device forwarding the transmission of the horse data to the monitor device. The transmission of the horse data to the monitor device may be performed using Bluetooth, for example by communicating with the monitor device directly over Bluetooth or via a Bluetooth gateway (the Bluetooth gateway may then be connected to a global network, such as the internet, for communicating with the monitor device), and/or over mobile network (such as 3G, 4G etc.).

Transmitting the horse data to the monitor device may for example comprise transmitting the horse data to a monitor device or gateway located proximate to the sensor device. The monitor device or gateway may be located within the range to be able to communicate with the sensor device when mounted on the horse, for example in or near a stable where the horse is located. The monitor device or gateway may then transmit the horse data to e.g. a server device (database) and/or a wireless electronic device, such as a mobile phone or smartphone of a user monitoring his horse, located remotely from the horse wearing the sensor device (for example the user being located at home), whereby the user may be informed on status of the horse.

In one or more exemplary methods, the first optical sensor is configured to emit infra-red electromagnetic radiation in the range from 700 nm to 1000 nm. To emit infra-red electromagnetic radiation in the range from 700 nm to 1000 nm may be understood as emitting infra-red electromagnetic radiation having a wavelength in the range from 700 nm to 1000 nm. In one or more exemplary methods, to emit infra-red electromagnetic radiation in the range from 700 nm to 1000 nm may comprise to emit infra-red electromagnetic radiation in the range from 750 nm to 1000 nm, preferably in the range 800 nm to 980 nm, more preferably in the range 900 nm to 960 nm, most preferably in the range 930 nm to 950 nm (such as 932 nm, 935 nm, 938 nm, 940 nm, 942 nm, 944 nm 946 nm, and/or 948 nm). The first optical sensor may comprise one or more LEDs configured to emit infra-red, IR, electromagnetic radiation in the range from 700 nm to 1000 nm. An advantage of emitting infra-red electromagnetic radiation in the range from 700 nm to 1000 nm, may be that the light emitted in that range may improve the penetration of the light in the fur and/or skin of the horse (for example compared to green light emitters), and in turn improving the measurement of the sensor device.

In one or more exemplary methods, positioning the sensor device below the ear of the horse comprises positioning (such as the user of the sensor device, for example the owner or the caretaker of the horse) the sensor device substantially at an artery of the horse.

In one or more exemplary methods and/or sensor devices, positioning the sensor device below the ear of the horse comprises positioning the sensor device at or near (such as within 5 cm from) a first portion of the artery of the horse. Thus, the sensor area may be representative of a first area located at the first portion of the artery.

In one or more exemplary methods and/or sensor devices, positioning the sensor device below the ear of the horse comprises positioning the sensor device at a second portion of the artery of the horse (such as the sensor area may be representative of a second area located at the second portion of the artery), and/or at a third portion of the artery of the horse (such as the sensor area may be representative of a third area located at the third portion of the artery), for example depending on the horse head equipment that the horse is wearing, such as where on the horse head equipment the sensor device may be attached and at which portion of the artery the sensor device is positioned (for example at which area the sensor device is positioned).

In one or more exemplary methods, the first optical sensor comprises a photoplethysmogram sensor. The photoplethysmogram sensor may be denoted as a PPG sensor. The PPG sensor may be configured to generate sensor data, such as first sensor data, for example based on measurements of the PPG sensor. The PPG sensor may be configured to optically obtain a plethysmogram, for example to detect blood volume changes in the microvascular bed of tissue of the horse. The PPG sensor may comprise a pulse oximeter configured to illuminate the fur/skin of the horse, e.g. with an IR light source, and to measure changes in light absorption (such as emitted or illuminated with the light from the optical source of the first optical sensor, for example from an LED).

In one or more exemplary methods, obtaining the sensor data comprises obtaining first heart rate data from the first optical sensor (such as from the PPG sensor). In one or more exemplary methods, obtaining the sensor data comprises obtaining the first sensor data comprising first heart rate data from the first optical sensor. The first heart rate data may comprise one or more electrical signals indicative of a heartbeat of the horse (such as an average heartbeat per minute), e.g. measured by the first optical sensor for a first time period. The first heart rate data may comprise a plurality of optical counts (such as measured by the first optical sensor, for example by the PPG sensor) over time which may be used to determine the horse data, such as used to determine a photoplethysmogram.

In one or more exemplary methods, determining the horse data comprises determining a photoplethysmogram, PPG based on the sensor data. In one or more exemplary methods, determining the horse data comprises determining a photoplethysmogram, PPG based on the first sensor data from the first sensor (such as from the PPG sensor, for example based on the first heart rate data).

In one or more exemplary methods and/or sensor devices, the second sensor comprises a motion sensor.

The motion sensor may for example comprise one or more of an accelerometer. The accelerometer may be or comprise a 3D motion sensor, for example a 3-axis, 12-bit digital accelerometer, and/or a gyroscope. The motion sensor may be configured to measure a motion of the horse or parts of the horse, such as configured to measure one or more movements of the horse, for example as second motion data indicative of a movement of the horse (e.g. accelerometer data indicative of an acceleration).

In one or more exemplary methods, the sensor data comprises second motion data from the motion sensor. In one or more exemplary methods, determining the horse data is based on the second motion data and the first sensor data. The second motion data may comprise data indicative of a movement of the horse (e.g. accelerometer data indicative of an acceleration). For example, the second motion data may comprise data indicative of the horse being active for example in a training situation, such as with the user and/or a rider. The horse being active may comprise one or more of walking, trotting, galloping, cantering, and pacing. For example, the second motion data may comprise data indicative of the horse being inactive, such as as standing still, resting and/or sleeping.

In one or more exemplary methods, determining the horse data comprises motion compensating (such as using the processor) the sensor data based on the second motion data. In one or more exemplary methods, determining the horse data comprises motion compensating the first sensor data based on the second motion data. In other words, determining the horse data may comprise to take a motion of the horse into account. In one or more exemplary methods, motion compensating the sensor data based on the second motion data comprises filtering noise in the sensor data obtained from the first optical sensor based on the second motion data (such as motion data of the horse). In one or more exemplary embodiments, motion compensating comprises to obtain (such as measure) the sensor data from the sensor unit over an extending period of time (for example obtaining sensor data comprising a longer measurement, such as to filter noise in the first sensor data due to motion of the horse).

In one or more exemplary methods, transmitting the horse data to the monitor device comprises transmitting the horse data according to a first transmission scheme. The first transmission scheme may be defined by first transmission settings, for example stored on a memory of the sensor device. The first transmission scheme may comprise or define one or more transmission cycles. The first transmission scheme may be defined during setup of the sensor device and/or updated via a first interface of the sensor device (such as a first wireless interface and/or a first wired interface). Accordingly, the sensor device may be configured to update the first transmission scheme in response to a settings update request via the interface of the sensor device. The first transmission scheme may be configured to adjust the sampling rate of the sensor data and/or the determination rate of horse data. The sampling rate may for example vary from in the range from 0.1 Hz to 200 Hz, such as from 1Hz to 10 Hz. The sampling rate may be determined based on a battery consumption of the sensor device (for example a higher sampling rate may be more battery consuming than a lower sampling rate). On the other hand, a higher sampling rate may provide a better sensitivity for the measurements of the sensor device. The transmission scheme may be configured such that the sensor device transmits to the monitor device in intervals, for example reducing the battery consumption of the sensor device. For example, the power efficiency improvements may be achieved by minimizing the data transmission, such as reporting based on activity and/or interval reporting. In other words, the first transmission scheme may comprise a reporting schedule. The reporting schedule may for example be implemented for reducing power consumption of the sensor device and in turn of the monitoring of the horse. The reporting schedule may comprise one or more schedules, such as a first schedule e.g. for training purpose, where the reporting may comprise a 1 second report (for example comprising one or more of heart rate, motion index, and gait type) for every second. The reporting schedule may comprise a second schedule e.g. for continuous long-term monitoring purpose, where the reporting may be done in intervals, e.g. once per minute the sensor device reports a 1-minute report, such as a 1-minute snapshot. The 1-minute report may be calculated for 1-minute measurements (such as observations), e.g. from 1-second reports. For example, the 1-minute report may be calculated based on one or more of a maximum, a 75% percentile, a median, a 25% percentile, and a minimum heart rate. For example, the 1-minute report may comprise a most frequent motion index and/or a most frequent gait measurement or observed in the 1-second reports. Furthermore, the 1-minute report may comprise one or more alarm flags, e.g. if there is a "mismatch" between the 1-minute median and the most frequent activity level (such as activity index). The reporting schedule may comprise a third schedule e.g. for long term spot measurements, where the reporting may be done in intervals with pauses in-between. For example, each 15 minutes interval 5 consecutive 1-minute reports are made. In other words, the third scheduling may be suitable for very long-term monitoring, e.g. a geofencing function i.e. monitoring that a horse is still there (theft prevention). The reporting schedule may thereby be adapted to the need of the user.

In one or more exemplary methods, transmitting the cardio data to the monitor device comprises transmitting the horse data in transmission events, wherein a time between consecutive transmission events is defined by the transmission scheme.

The first transmission scheme may define a transmission cycle frequency or time between two consecutive first transmission cycles. A transmission cycle frequency may be once per 10 seconds, once per 20 seconds, once per 30 seconds, once per minute, once per 2 minutes, once per 5 minutes, once per 10 minutes, once per 20 minutes, once per 30 minutes or less. A time between two consecutive first transmission cycles may be in the range from 10 seconds to several minutes, such as 30 seconds, 1 minute, 2 minutes, or more, such as 5 minutes, 10 minutes, 20 minutes or 30 minutes.

In one or more exemplary methods, the method comprising determining an activity level indicative of physical activity of the horse based on the sensor data. The activity level of the horse may be comprised in the horse data, e.g. as a motion index. The activity level of the horse may be determined based on the second sensor data. The activity level of the horse may comprise one or more activity levels, such as one or more of a first level of activity indicative of a first level of activity of the horse, a second level of activity indicative of a second level of activity of the horse, a third level of activity indicative of a third level of activity of the horse, a fourth level of activity indicative of a fourth level of activity of the horse, and/or more levels of activity) of the horse indicative of a level of activity of the horse, for example to indicate different states of activity of the horse. The activity level of the horse may be determined based on the sensor data, such as the second sensor data, such as the second motion data, for example based on the sensor data being above and/or below one or more thresholds. For example, the first level of activity may correspond to an activity level above or below a first threshold, the second level of activity may correspond to an activity level above or below a second threshold or within a second range, the third level of activity may correspond to an activity level above or below a third threshold or within a third range, and/or the fourth level of activity may correspond to an activity level above or below a fourth threshold or within a fourth range. The first threshold may be below the second threshold which may be below the third threshold which may be below the fourth threshold or vice versa. In other words, the activity level of the horse may thereby be determined (such as measured) on a scale e.g. based on the one or more thresholds. For example, the activity level (such as physical activity level) may comprise a number on a scale between 0 and 4, such as 0 being indicative of very low activity (such as no activity, for example sleeping or resting), 1 being indicative of low activity, 2 being indicative of medium activity, 3 being indicative of medium-high activity, and 4 being indicative of high activity.

In one or more embodiments, to determine the activity level of the horse may comprise to determine a gait type of the horse. For example, the gait type of the horse may be determined based on the second sensor data, such as based on the second motion data (e.g. based on an analysis of the second motion data, such as 3D motion sensor data). For example, the activity level (such as gait type) may comprise a number on a scale between 0 and 4, such as 0 being indicative of very low activity (such as no activity, for example sleeping or resting), 1 being indicative of walk (such as four beat movement, e.g. low speed, such as 3 to 5 mph), 2 being indicative of trot (such as two beat movement, e.g. medium to high speed, such as 6 to 10 mph), 3 being indicative of canter (such as three beat movement, e.g. medium to high speed, such as 8 to 12 mph), and 4 being indicative of gallop (such as 4 beat movement, e.g. high speed, such as 20 to 35 mph).

In one or more exemplary methods, the transmission of the horse data to the monitor device is based on the activity level and/or cardiac data, such as heart rate. The transmission of horse data may be dependent on detection of a mismatch between the motion data, such as activity level, and the cardiac data, such as heart rate.

Thus, the transmission of the horse data to the monitor device may be motion dependent, such as motion dependent transmission. For example, the sensor device may be configured to transmit the horse data when the activity level is above or below a threshold. In other words, the sensor device may be configured to transmit the horse data when the horse is active (i.e. not resting or sleeping). In one or more embodiments, the activity level of the horse may trigger the transmission of the horse data to the monitor device.

A sensor device is disclosed. The sensor device comprises a sensor device housing, a memory, a processor, and a sensor unit comprising a first optical sensor connected to the processor. The sensor device is configured to be positioned below an ear of a horse. The processor is configured to obtain sensor data from the sensor unit, the sensor data comprising first sensor data from the first optical sensor. The processor is configured to determine horse data of the horse based on the sensor data. The processor is configured to transmit the horse data to a monitor device.

Further, a horse head gear is disclosed, the horse head gear comprising a horse head equipment and a sensor device according to this disclosure. The horse head gear may comprise horse head equipment such as one or more of a bridle, a harness, and a head collar.

Further, a monitoring system is disclosed, the monitoring system (such as remote wireless monitoring system) comprising a sensor device according to this disclosure and a monitor device for monitoring the sensor device.

In one or more embodiments, the sensor device is embedded in the horse head equipment, such as embedded in one or more of a bridle, a harness, and a head collar of the horse.

It is to be understood that a description of a feature in relation to sensor device(s) is also applicable to the corresponding method(s) and vice versa.

Fig. 1 is a schematic representation illustrating an example scenario of a sensor device 300 according to the disclosure, positioned below an ear of a horse 1. The sensor device 300 is transmitting 14 horse data to a monitor device 400. For example, a user (such as a horse owner, a horse trainer, a horse rider, or a person taking care of the horse) may monitor the horse 1 on the monitor device 400 by receiving horse data on the monitor device. The user monitoring the horse 1 may for example be able to receive information about the horse 1 during a training of the horse 1 (such as while riding the horse or on the ground next to the horse) or while the horse is in the stable (such as monitoring a level of activity of the horse during the day or the night, a stress level of the horse, a health condition of the horse, whether the horse is sexually receptive, pregnant, or in gestation). The sensor device may transmit (such as report) to the monitor device, for notifying the owner or caregiver to attend the distressed horse. For example, the horse may comprise a heart rate of the horse, which may be indicative (such as a key indicator) of distress of the horse (such as foaling), e.g. if the horse has a unusually high heart rate (for example an unusually high heart rate when being in the stall, or at an unusual time).

On Fig. 1 the sensor device 300 is positioned in a sensor area 10. The sensor area 10 may be representative of an area below the ear of the horse 1 where the sensor device 300 may be positioned. In Fig. 1, the sensor device 300 is shown for illustrative purposes above (such as on top of) a horse head equipment 12. In practice (such as in use, for example a mounted state of the sensor device), the sensor device 300 may be positioned below the horse head equipment 12 (for example removably attached to the horse head equipment 12, e.g. with an attachment element such as a strap) in contact with a proximal surface, such as a skin surface of the horse.

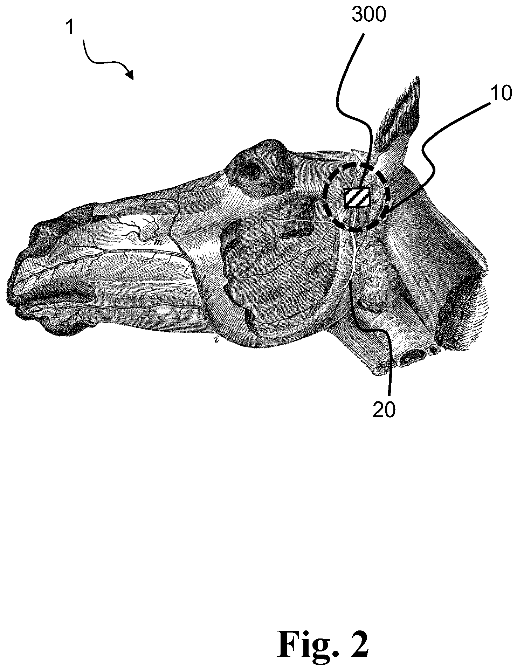

Fig. 2 is a schematic representation illustrating an example scenario of a sensor device 300 according to the disclosure, positioned below an ear of a horse 1 (such as at a sensor area 10). In Fig. 2, the sensor area 10 is located at a first portion of an artery 20 of the horse 1 (such as the sensor area 10 may be representative of a first area located at the first portion of the artery 20). In one or more exemplary methods and/or sensor devices, the artery 20 may be an arteria temporalis superficialis of the horse 1. In one or more exemplary methods and/or sensor devices, the sensor device 300 may be configured to be positioned at a second portion of the artery 20 of the horse 1 (such as the sensor area 10 may be representative of a second area located at the second portion of the artery 20), and/or at a third portion of the artery 20 of the horse 1 (such as the sensor area 10 may be representative of a third area located at the third portion of the artery 20), for example depending on the horse head equipment that the horse is wearing, such as where on the horse head equipment the sensor device 300 may be attached and at which portion of the artery 20 the sensor device 300 is positioned (for example at which area the sensor device 300 is positioned).

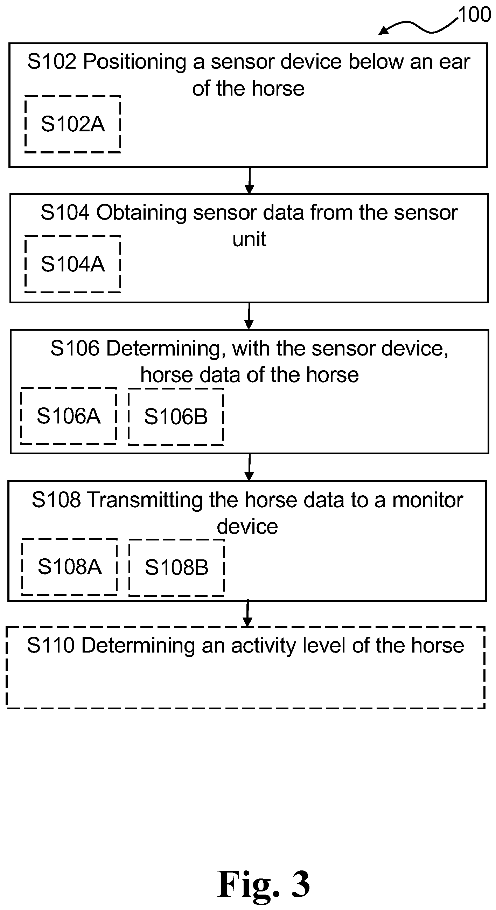

Fig. 3 is a flow-chart illustrating an example method 100, for monitoring a horse (such as performed by the sensor device disclosed herein, such as sensor device 300 of Figs. 1, 2, 4, and 5A-5B), according to this disclosure.

The method 100 comprises positioning S102 a sensor device below an ear of the horse, the sensor device comprising a sensor unit comprising a first optical sensor. In one or more methods and/or sensor devices, positioning the sensor device below an ear of the horse comprises to position the sensor device in a sensor area. The sensor area may be representative of an area below the ear of the horse where the sensor device may be positioned. The term below the ear of the horse may be understood as between the ear of the horse and a temporal bone of the horse, e.g. substantially at the skin surface where an arteria temporalis superficialis is located under the skin. The area below the ear of the horse may be rich in arterial blood (for example close to the arteria temporalis superficialis).

Most horses have a natural recess below the ear at the temporal (above the temporal bone), where it may be advantageous to position the sensor device (for example such that the sensor device may be stabilized, e.g. improving the measurement of the sensor device).

The method 100 comprises obtaining S104 sensor data from the sensor unit, the sensor data comprising first sensor data from the first optical sensor. The first sensor data may comprise and/or be based on one or more first optical signals (such as one or more optical counts measured by the first optical sensor over time). The first optical sensor may comprise one or more optical sources (such as one or more transmitters or emitters of optical signals, for example photo emitters, e.g. LEDs photodiodes), and/or one or more optical receivers (such as photo detectors). The first optical sensor/sensor unit may be configured to measure a heart-rate, a cardiac cycle, a respiration, and/or a blood pressure of the horse.

In one or more exemplary methods, the sensor unit comprises a second sensor, and the sensor data comprises second sensor data from the second sensor.

The method 100 comprises determining S106 (such as using the processor), with the sensor device, horse data of the horse based on the sensor data. In one or more exemplary methods, determining horse data of the horse based on the sensor data, may comprise determining the horse data based on the first sensor data. For example, determining the horse data may comprise determining a heart rate (such as a pulse of the horse) of the horse based on one or more first optical signals (such as one or more optical counts measured by the first optical sensor over time). For example, determining the horse data may comprise determining a heart-rate, a cardiac cycle, a pulse, a respiration, and/or a blood pressure of the horse. In one or more embodiments, determining the horse data comprises to process the sensor data. For example, the processor may be configured to integrate one or more the sensor data to obtain one derived measurement..

The method 100 comprises transmitting S108, e.g. via the first interface, the horse data to a monitor device. The monitor device, such as the monitor device 400 of Fig. 1, may comprise one or more of: an electronic device (such as a wireless electronic device), a mobile phone, a smartphone, and/or a computer. The transmission of the horse data may be performed directly to the monitor device or may occur through a relay device forwarding the transmission of the horse data to the monitor device. The transmission of the horse data to the monitor device may be performed using Bluetooth, for example by communicating with the monitor device directly over Bluetooth or via a Bluetooth gateway (the Bluetooth gateway may then be connected to a global network, such as the internet, for communicating with the monitor device), and/or over mobile network (such as 3G, 4G etc.).

Transmitting the horse data to the monitor device may for example comprise transmitting the horse data to a monitor device or gateway located proximate to the sensor device. The monitor device or gateway may be located within the range to be able to communicate with the sensor device when mounted on the horse, for example in or near a stable where the horse is located). The monitor device or gateway may then transmit the horse data to e.g. a server device (database) and/or a wireless electronic device, such as a mobile phone or smartphone of a user monitoring his horse, located remotely from the horse wearing the sensor device (for example the user being located at home), whereby the user may be informed on status of the horse.

In one or more exemplary methods, the first optical sensor is configured to emit infra-red electromagnetic radiation in the range from 700 nm to 1000 nm. To emit infra-red electromagnetic radiation in the range from 700 nm to 1000 nm may be understood as emitting infra-red electromagnetic radiation having a wavelength in the range from 700 nm to 1000 nm. In one or more exemplary methods, to emit infra-red electromagnetic radiation in the range from 700 nm to 1000 nm may comprise to emit infra-red electromagnetic radiation in the range from 750 nm to 1000 nm, preferably in the range 800 nm to 980 nm, more preferably in the range 900 nm to 960 nm, most preferably in the range 930 nm to 950 nm (such as 932 nm, 935 nm, 938 nm, 940 nm, 942 nm, 944 nm 946 nm, and/or 948 nm). The first optical sensor may comprise one or more LEDs configured to emit infra-red, IR, electromagnetic radiation in the range from 700 nm to 1000 nm. An advantage of emitting infra-red electromagnetic radiation in the range from 700 nm to 1000 nm, may be that the light emitted in that range may improve the penetration of the light in the fur and/or skin of the horse (for example compared to green light emitters), and in turn improving the measurement.

In one or more exemplary methods, positioning S102 the sensor device below the ear of the horse comprises positioning S102A (such as the user of the sensor device, for example the owner or the caretaker of the horse) the sensor device substantially at an artery of the horse.

In one or more exemplary methods and/or sensor devices, positioning the sensor device below the ear of the horse comprises positioning the sensor device at or near (such as within 5 cm from) a first portion of the artery of the horse. Thus, the sensor area may be representative of a first area located at the first portion of the artery.

In one or more exemplary methods and/or sensor devices, positioning the sensor device below the ear of the horse comprises positioning the sensor device at a second portion of the artery of the horse (such as the sensor area may be representative of a second area located at the second portion of the artery), and/or at a third portion of the artery of the horse (such as the sensor area may be representative of a third area located at the third portion of the artery), for example depending on the horse head equipment that the horse is wearing, such as where on the horse head equipment the sensor device may be attached and at which portion of the artery the sensor device is positioned (for example at which area the sensor device is positioned).

In one or more exemplary methods, the first optical sensor comprises a photoplethysmogram sensor. The photoplethysmogram sensor may be denoted as a PPG sensor. The PPG sensor may be configured to generate sensor data, such as first sensor data, for example based on measurements of the PPG sensor. The PPG sensor may be configured to optically obtain a plethysmogram, for example to detect blood volume changes in the microvascular bed of tissue of the horse. The PPG sensor may comprise a pulse oximeter configured to illuminate the skin of the horse and to measure changes in light absorption (such as emitted or illuminated with the light from the optical source of the first optical sensor, for example from an LED).

In one or more exemplary methods, obtaining S104 the sensor data comprises obtaining S104A first heart rate data from the first optical sensor (such as from the PPG sensor). In one or more exemplary methods, obtaining S104 the sensor data comprises obtaining S104A the first sensor data comprising first heart rate data from the first optical sensor. The first heart rate data may comprise one or more electrical signals indicative of a heartbeat of the horse (such as an average heartbeat per minute), e.g. measured by the first optical sensor for a first time period. The first heart rate data may comprise a plurality of optical counts (such as measured by the first optical sensor, for example by the PPG sensor) over time which may be used to determine the horse data, such as used to determine a photoplethysmogram.

In one or more exemplary methods, determining S106 the horse data comprises determining S106A a photoplethysmogram, PPG based on the sensor data. In one or more exemplary methods, determining S106 the horse data comprises determining S106A a photoplethysmogram, PPG based on the first sensor data from the first sensor (such as from the PPG sensor, for example based on the first heart rate data).

In one or more exemplary methods, the second sensor comprises a motion sensor.

The motion sensor may for example comprise one or more of an accelerometer (such as a 3D motion sensor, for example a 3-axis, 12-bit digital accelerometer) and a gyroscope. The motion sensor may be configured to measure a motion of the horse, such as configured to measure one or more movements of the horse, for example as second motion data indicative of a movement of the horse (e.g. accelerometer data indicative of an acceleration).

In one or more exemplary methods, the sensor data comprises second motion data from the motion sensor. In one or more exemplary methods, determining S106 the horse data is based on the second motion data and the first sensor data. The second motion data may comprise data indicative of a movement of the horse (e.g. accelerometer data indicative of an acceleration). For example, the second motion data may comprise data indicative of the horse being active (e.g. in a training situation, such as with the user and/or a rider) such as walking, trotting, galloping, cantering and/or pacing or inactive such as standing still, resting and/or sleeping.

In one or more exemplary methods, determining S106 the horse data comprises motion compensating S106B (such as using the processor) the sensor data based on the second motion data. In one or more exemplary methods, determining S106 the horse data comprises motion compensating S106B the first sensor data based on the second motion data. In other words, determining the horse data may comprise to take a motion of the horse into account. In one or more exemplary methods, motion compensating S106B the sensor data based on the second motion data comprises filtering noise in the sensor data obtained from the first optical sensor based on the second motion data (such as motion data of the horse). In one or more exemplary embodiments, motion compensating comprises to obtain (such as measure) the sensor data from the sensor unit over an extending period of time (for example obtaining sensor data comprising a longer measurement, such as to filter noise in the first sensor data due to motion of the horse).

In one or more exemplary methods, transmitting S108 the horse data to the monitor device comprises transmitting S108A the horse data according to a first transmission scheme. The first transmission scheme may be defined by first transmission settings, for example stored on a memory of the sensor device. The first transmission scheme may comprise one or more transmission cycles. The first transmission scheme may be defined during setup of the sensor device and/or updated via a first interface of the sensor device (such as a first wireless interface and/or a first wired interface). Accordingly, the sensor device may be configured to update the first transmission scheme in response to a settings update request via the interface of the sensor device. The first transmission scheme may be configured to adjust the sampling rate of the sensor data and/or the determination rate of horse data. The sampling rate may for example vary from in the range from 0.1 Hz to 200 Hz, such as from 1Hz to 10 Hz. The sampling rate may be determined based on a battery consumption of the sensor device (for example a higher sampling rate may be more battery consuming than a lower sampling rate). On the other hand, a higher sampling rate may provide a better sensitivity for the measurements of the sensor device. The transmission scheme may be configured such that the sensor device transmits to the monitor device in intervals, for example reducing the battery consumption of the sensor device. For example, the power efficiency improvements may be achieved by minimizing and/or adapting the data transmission, such as reporting based on activity and/or interval reporting. In other words, the first transmission scheme may comprise a reporting schedule. The reporting schedule may for example be implemented for reducing power consumption of the sensor device and in turn of the monitoring of the horse. The reporting schedule may comprise one or more schedules, such as a first schedule e.g. for training purpose, where the reporting may comprise a 1 second report (for example comprising one or more of heart rate, motion index, and gait type) for every second. The reporting schedule may comprise a second schedule e.g. for continuous long-term monitoring purpose, where the reporting may be done in intervals, e.g. once per minute the sensor device reports a 1-minute report, such as a 1-minute snapshot. The 1-minute report may be calculated for 1-minute measurements (such as observations), e.g. from 1-second reports. For example, the 1-minute report may be calculated based on one or more of a maximum, a 75% percentile, a median, a 25% percentile, and a minimum heart rate. For example, the 1-minute report may comprise a most frequent motion index and/or a most frequent gait measurement or observed in the 1-second reports. Furthermore, the 1-minute report may comprise one or more alarm flags, e.g. if there is a "mismatch" between the 1-minute median and the most frequent activity level (such as activity index). The reporting schedule may comprise a third schedule e.g. for long term spot measurements, where the reporting may be done in intervals with pauses in-between. For example, each 15 minutes interval 5 consecutive 1-minute reports are made. In other words, the third scheduling may be suitable for very long-term monitoring, e.g. a geofencing function i.e. monitoring that a horse is still there (theft prevention). The reporting schedule may thereby be adapted to the need of the user.

In one or more exemplary methods, transmitting S108 the cardio data to the monitor device comprises transmitting S108B the horse data in transmission events, wherein a time between consecutive transmission events is defined by the transmission scheme.

The first transmission scheme may define a transmission cycle frequency or time between two consecutive first transmission cycles. A transmission cycle frequency may be once per 10 seconds, once per 20 seconds, once per 30 seconds, once per minute, once per 2 minutes, once per 5 minutes, once per 10 minutes, once per 20 minutes, once per 30 minutes or less. A time between two consecutive first transmission cycles may be in the range from 10 seconds to several minutes, such as 30 seconds, 1 minute, 2 minutes, or more, such as 5 minutes, 10 minutes, 20 minutes or 30 minutes.

In one or more exemplary methods, the method comprising determining S110 an activity level of the horse based on the sensor data. The activity level of the horse may be comprised in the horse data. The activity level of the horse may be determined based on the second sensor data. The activity level of the horse may comprise one or more activity levels (such as a first level of activity indicative of a first level of activity of the horse, a second level of activity indicative of a second level of activity of the horse, a third level of activity indicative of a third level of activity of the horse, a fourth level of activity indicative of a fourth level of activity of the horse, and/or more levels of activity) of the horse indicative of a level of activity of the horse, for example to indicate different states of activity of the horse. The activity level of the horse may be determined based on the sensor data (such as the first sensor data and/or the second sensor data, for example based on the first heart rate data and/or the second motion data) being above or below one or more thresholds. For example, the first level of activity may be above or below a first threshold, the second level of activity may be above or below a second threshold, the third level of activity may be above or below a third threshold, and/or the fourth level of activity may be above or below a fourth threshold. The first threshold may be below the second threshold which may be below the third threshold which may be below the fourth threshold or vice versa. In other words, the activity level of the horse may thereby be determined (such as measured) on a scale e.g. based on the one or more thresholds. For example, the activity level (such as physical activity level) may comprise a number on a scale between 0 and 4, such as 0 being indicative of very low activity (such as no activity, for example sleeping or resting), 1 being indicative of low activity, 2 being indicative of medium activity, 3 being indicative of medium-high activity, and 4 being indicative of high activity.

In one or more embodiments, to determine the activity level of the horse may comprise to determine a gait type of the horse. For example, the gait type of the horse may be determined based on the second sensor data, such as based on the second motion data (e.g. based on an analysis of the second motion data, such as 3D motion sensor data). For example, the activity level (such as gait type) may comprise a number on a scale between 0 and 4, such as 0 being indicative of very low activity (such as no activity, for example sleeping or resting), 1 being indicative of walk (such as four beat movement, e.g. low speed, such as 3 to 5 mph), 2 being indicative of trot (such as two beat movement, e.g. medium to high speed, such as 6 to 10 mph), 3 being indicative of canter (such as three beat movement, e.g. medium to high speed, such as 8 to 12 mph), and 4 being indicative of gallop (such as 4 beat movement, e.g. high speed, such as 20 to 35 mph).

In one or more exemplary methods, the transmission S108 of the horse data to the monitor device is based on the activity level. In other words, the transmission of the horse data to the monitor device may be motion dependent, such as motion dependent transmission. For example, the sensor device may be configured to transmit the horse data when the activity level is above or below a threshold. In other words, the sensor device may be configured to transmit the horse data when the horse is active (i.e. not resting or sleeping). In one or more embodiments, the activity level of the horse may trigger the transmission of the horse data to the monitor device.

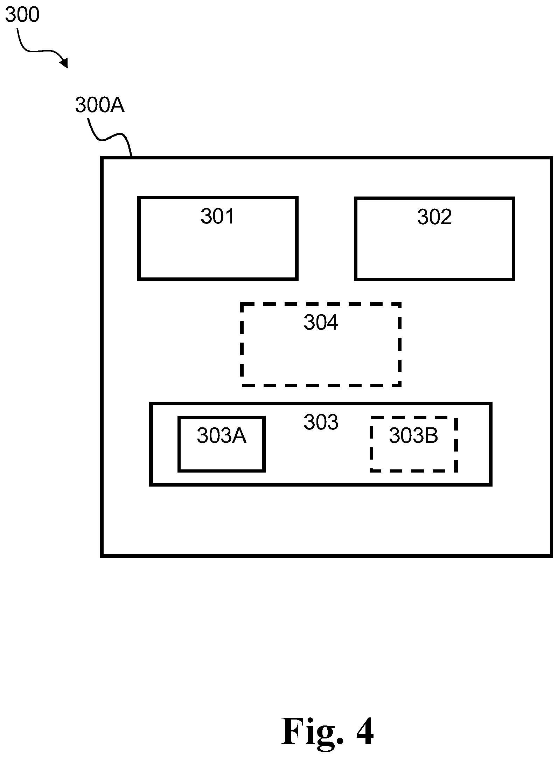

Fig. 4 shows a block diagram illustrating a sensor device 300 according to the disclosure.

The sensor device 300 comprises a sensor device housing 300A, a memory 301, a processor 302 (such as a first processor), and a sensor unit 303 comprising a first optical sensor 303A connected to the processor 302. The sensor device 300 may comprise a battery (not shown). The sensor device 300 is configured to be positioned below an ear of a horse. The processor 302 is configured to obtain sensor data from the sensor unit 303, the sensor data comprising first sensor data from the first optical sensor 303A. The processor 302 is configured to determine horse data of the horse based on the sensor data. The processor 302 is configured to transmit the horse data to a monitor device.

In one or more embodiments, the sensor device 300 comprises a first processor and a second processor. For example, the first processor may be a high frequency processor (such as a processor operating at a high frequency) and the second processor may be low frequency processor (such as a processor operating at a low frequency, for example lower than the processor operating at high frequency).

In one or more exemplary sensor devices, the sensor unit 303 comprises a second sensor 303B. In one or more exemplary sensor devices, the sensor data comprises second sensor data from the second sensor 303B.

In one or more exemplary sensor devices, the sensor device 300 comprises a first interface 304. The first interface 304 may comprise one or more first primary interfaces (such as one or more wireless interfaces, for example one or more antennas, such as an antenna for using short range wireless communication Bluetooth low energy transmission to the monitor device) and/or one or more first secondary interfaces (such as one or more wired interfaces, for example a USB interface).

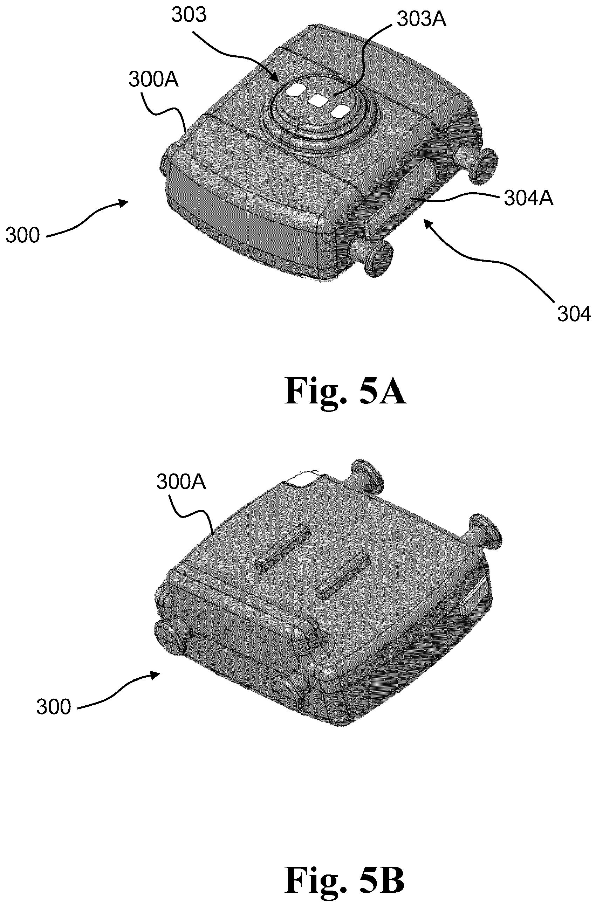

Figs. 5A-5B are perspective views of an example sensor device according to the disclosure seen respectively from the proximal side and the distal side.

Fig. 5A is a perspective view of the sensor device 300 seen from the proximal side and Fig. 5B is a perspective view of the sensor device 300 seen from the distal side. The sensor device 300 comprises a sensor device housing 300A and a sensor unit 303 comprising a first optical sensor 303A. The sensor device 300 is configured to be positioned below an ear of a horse. The first optical sensor 303A is configured to face the proximal surface (such as to be substantially in contact with the horse fur).

In one or more exemplary sensor devices, the sensor unit 303 comprises a second sensor 303B (not shown).

In one or more exemplary sensor devices, the sensor device 300 comprises a first interface 304. The first interface 304 may comprise one or more first primary interfaces (such as one or more wireless interfaces) and/or one or more first secondary interfaces (such as one or more wired interfaces). In the shown embodiment, the first interface 304 comprises a wired interface comprising one or more connectors (such as terminals) and comprising an interface cover 304A (such as a closing element for the interface connector). The interface cover 304A may for example comprise a rubber plug, e.g. for providing a tight seal (such as sealing the first interface when the interface cover is in the mounted state). In one or more embodiments, the first interface comprises a USB connector (such as a USB terminal), for example comprising a USB cover as the interface cover.

The sensor device 300 may be configured to perform any of the methods disclosed in Fig. 3. In other words, the sensor device 300 may be configured for monitoring a horse.

The processor circuitry 302 is optionally configured to perform any of the operations disclosed in Fig. 3 (such as any one or more of S102A, S104A, S106A, S106B, S108A, S108B, S110). The operations of the sensor device 300 may be embodied in the form of executable logic routines (for example, lines of code, software programs, etc.) that are stored on a non-transitory computer readable medium (for example, memory circuitry 301) and are executed by the processor circuitry 302).
Furthermore, the operations of the sensor device 300 may be considered a method that the sensor device 300 is configured to carry out. Also, while the described functions and operations may be implemented in software, such functionality may as well be carried out via dedicated hardware or firmware, or some combination of hardware, firmware and/or software.

Memory circuitry 301 may be one or more of a buffer, a flash memory, a hard drive, a removable media, a volatile memory, a non-volatile memory, a random access memory (RAM), or other suitable device. In a typical arrangement, memory circuitry 301 may include a non-volatile memory for long term data storage and a volatile memory that functions as system memory for processor circuitry 302. Memory circuitry 301 may exchange data with processor circuitry 302 over a data bus. Control lines and an address bus between memory circuitry 301 and processor circuitry 302 also may be present (not shown in Fig. 2). Memory circuitry 301 is considered a non-transitory computer readable medium.

Memory circuitry 301 may be configured to store information (such as information indicative of the sensor data, the horse data, the first sensor data, the second sensor data, the first heart rate data, the second motion data, the PPG) in a part of the memory.

It is to be understood that a description of a feature in relation to sensor device(s) is also applicable to the corresponding method(s), and vice versa.

Disclosed are methods, sensor device and monitoring system according to any of the following items.
Item 1. A method for monitoring a horse, the method comprising:
   - positioning (S102) a sensor device below an ear of the horse, the sensor device comprising a sensor unit comprising a first optical sensor;
   - obtaining (S104) sensor data from the sensor unit, the sensor data comprising first sensor data from the first optical sensor;
   - determining (S106), with the sensor device, horse data of the horse based on the sensor data; and
   - transmitting (S108) the horse data to a monitor device.
Item 2. The method according to item 1, wherein the sensor unit comprises a second sensor and wherein the sensor data comprises second sensor data from the second sensor.
Item 3. The method according to any of items 1-2, wherein the first optical sensor is configured to emit infra-red electromagnetic radiation in the range from 700 nm to 1000 nm.
Item 4. The method according to any of items 1-3, wherein positioning (S102) the sensor device below the ear of the horse comprises positioning (S102A) the sensor device substantially at an artery of the horse.
Item 5. The method according to any of items 1-4, wherein the first optical sensor comprises a photoplethysmogram sensor.
Item 6. The method according to any of items 1-5, wherein obtaining (S104) the sensor data comprises obtaining (S104A) first heart rate data from the first optical sensor.
Item 7. The method according to any of items 2-6 as dependent on item 2, wherein the second sensor comprises a motion sensor.
Item 8. The method according to item 7, wherein the sensor data comprises second motion data from the motion sensor.
Item 9. The method according to any of items 1-8, wherein determining (S106) the horse data comprises determining (S106A) a photoplethysmogram, PPG, based on the sensor data.
Item 10. The method according to any of items 8-9, wherein determining (S106) the horse data comprises motion compensating (S106B) the sensor data based on the second motion data.
Item 11. The method according to any of items 1-10, wherein transmitting (S108) the horse data to the monitor device comprises transmitting (S108A) the horse data according to a first transmission scheme.
Item 12. The method according to item 11, wherein transmitting (S108) the cardio data to the monitor device comprises transmitting (S108B) the horse data in transmission events, wherein a time between consecutive transmission events is defined by the transmission scheme.
Item 13. The method according to any of items 1-12, the method comprising determining (S110) an activity level of the horse based on the sensor data.
Item 14. The method according to item 13, wherein the transmission (S108) of the horse data to the monitor device is based on the activity level.
Item 15. A sensor device configured to operate according to the method of any of items 1-14.
Item 16. A sensor device, comprising:
   - a sensor device housing;
   - a memory;
   - a processor;
   - a sensor unit comprising a first optical sensor connected to the processor;
   - wherein the sensor device is configured to be positioned below an ear of a horse,
   wherein the processor is configured to:
   - obtain sensor data from the sensor unit, the sensor data comprising first sensor data from the first optical sensor;
   - determine horse data of the horse based on the sensor data; and
   - transmit the horse data to a monitor device.
Item 17. Sensor device according to item 16, wherein the sensor unit comprises a second sensor and wherein the sensor data comprises second sensor data from the second sensor.
Item 18. A horse head gear comprising a horse head equipment and a sensor device according to any of items 16-17.
Item 19. A monitoring system comprising a sensor device according to any of items 16-17 and a monitor device for monitoring the sensor device.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa. It may be appreciated that Figs. 1-2, 4, and 5 comprise some modules or operations which are illustrated with a solid line and some modules or operations which are illustrated with a dashed line. The modules or operations which are comprised in a solid line are modules or operations which are comprised in the broadest example embodiment. The modules or operations which are comprised in a dashed line are example methods and/or sensor devices which may be comprised in, or a part of, or are further modules or operations which may be taken in addition to the modules or operations of the solid line example methods and/or sensor devices. It should be appreciated that these operations need not be performed in order presented. Furthermore, it should be appreciated that not all of the operations need to be performed. The exemplary operations may be performed in any order and in any combination.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary methods and/or sensor devices may be implemented at least in part by means of both hardware and software, and that several "means", "units" or "devices" may be represented by the same item of hardware.

The various exemplary methods and devices described herein are described in the general context of method steps processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly, to be regarded in an illustrative rather than restrictive sense. The claimed invention is intended to cover all alternatives, modifications, and equivalents.

### LIST OF REFERENCES

1 horse
10 sensor area
12 horse head equipment
14 transmitting
20 artery
300 sensor device
300A sensor device housing
301 memory
302 processor
303 sensor unit
303A first optical sensor
303B second sensor
304 first interface
400 monitoring device
100 method for monitoring a horse
S102 positioning a sensor device below an ear of the horse, the sensor device comprising a sensor unit comprising a first optical sensor
S102A positioning the sensor device substantially at an artery of the horse
S104 obtaining sensor data from the sensor unit, the sensor data comprising first sensor data from the first optical sensor
S104A obtaining first heart rate data from the first optical sensor
S106 determining, with the sensor device, horse data of the horse based on the sensor data
S106A determining a photoplethysmogram, PPG, based on the sensor data
S106B motion compensating the sensor data based on the second motion data
S108 transmitting the horse data to a monitor device
S108A transmitting the horse data according to a first transmission scheme
S108B transmitting the horse data in transmission events, wherein a time between consecutive transmission events is defined by the transmission scheme
S110 determining an activity level of the horse based on the sensor data

## Claims

1. A method for monitoring a horse, the method comprising:
- positioning a sensor device below an ear of the horse, the sensor device comprising a sensor unit comprising a first optical sensor;
- obtaining sensor data from the sensor unit, the sensor data comprising first sensor data from the first optical sensor;
- determining, with the sensor device, horse data of the horse based on the sensor data; and
- transmitting the horse data to a monitor device.

2. The method according to claim 1, wherein the sensor unit comprises a second sensor and wherein the sensor data comprises second sensor data from the second sensor.

3. The method according to any of the preceding claims, wherein the first optical sensor is configured to emit infra-red electromagnetic radiation in the range from 700 nm to 1000 nm.

4. The method according to any of the preceding claims, wherein positioning the sensor device below the ear of the horse comprises positioning the sensor device substantially at an artery of the horse.

5. The method according to any of the preceding claims, wherein the first optical sensor comprises a photoplethysmogram sensor.

6. The method according to any of the preceding claims, wherein obtaining the sensor data comprises obtaining first heart rate data from the first optical sensor.

7. The method according to any of claims 2-6 as dependent on claim 2, wherein the second sensor comprises a motion sensor.

8. The method according to claim 7, wherein the sensor data comprises second motion data from the motion sensor.

9. The method according to any of the preceding claims, wherein determining (S106) the horse data comprises determining a photoplethysmogram, PPG, based on the sensor data.

10. The method according to claim 8, wherein determining the horse data comprises motion compensating the sensor data based on the second motion data.

11. The method according to any of the preceding claims, wherein transmitting the horse data to the monitor device comprises transmitting the horse data according to a first transmission scheme.

12. The method according to claim 11, wherein transmitting the cardio data to the monitor device comprises transmitting the horse data in transmission events, wherein a time between consecutive transmission events is defined by the transmission scheme.

13. The method according to any of the preceding claims, the method comprising determining an activity level of the horse based on the sensor data.

14. A sensor device, comprising:
- a sensor device housing;
- a memory;
- a processor;
- a sensor unit comprising a first optical sensor connected to the processor;
- wherein the sensor device is configured to be positioned below an ear of a horse,
wherein the processor is configured to:
- obtain sensor data from the sensor unit, the sensor data comprising first sensor data from the first optical sensor;
- determine horse data of the horse based on the sensor data; and
- transmit the horse data to a monitor device.

15. Sensor device according to claim 14, wherein the sensor unit comprises a second sensor and wherein the sensor data comprises second sensor data from the second sensor.
